# EUROPEAN PATENT APPLICATION

(11) **EP 4 248 950 A1**
(43) Date of publication of application: **27.09.2023**
(21) Application number: 21898701.4
(22) Date of filing: 29.11.2021
(51) Int. Cl.: A61K 9/16, A61K 9/00, A61K 31/4725, A61P 19/02, A61P 29/00

(54) **INJECTABLE COMPOSITION CONTAINING CASPASE INHIBITOR, AND PREPARATION METHOD THEREFOR**

(30) Priority: 30.11.2020 KR 20200164625
(71) Applicant: LG Chem, Ltd., Seoul 07336 (KR)
(72) Inventor: YOO, Seok Cheol, Cheongju-si Chungcheongbuk-do 28162 (KR); KIM, Sung Won, Seoul 07796 (KR); PARK, Jung Gyu, Seoul 07796 (KR); CHOI, Sei Hyun, Seoul 07796 (KR); PARK, Hee Dong, Seoul 07796 (KR); BAEK, Jae Uk, Seoul 07796 (KR)
(74) Representative: Hoffmann Eitle
(86) International application number: PCT/KR2021/017717
(87) International publication number: WO 2022/114876

(57) **Abstract**

The present invention relates to an injectable pharmaceutical composition containing a caspase inhibitor, and a preparation method therefor, and, more specifically, to an injectable pharmaceutical composition, and a preparation method therefor, the composition comprising: as an active ingredient, (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, which is a caspase inhibitor, or a pharmaceutically acceptable salt or isomer thereof; and, as a biocompatible polymer, microspheres containing poly(lactide-co-glycolide).

## Description

### TECHNICAL FIELD

The present invention relates to a pharmaceutical composition for injection comprising a caspase inhibitor and a method of preparation therefor. More specifically, the present invention relates to a pharmaceutical composition for injection comprising a microsphere which comprises (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof as an active ingredient; and poly(lactide-co-glycolide) as a biocompatible polymer, and a method of preparation therefor.

### BACKGROUND ART

Caspases are a type of enzymes and are cysteine proteases that exist as an α2β2 tetramer. Caspase inhibitors interfere with the activity of these caspases, thereby regulating inflammation or apoptosis caused by the action of caspases. Diseases in which symptoms can be eliminated or alleviated by administration of these compounds include osteoarthritis, rheumatoid arthritis, degenerative arthritis, destructive bone disorder, hepatic diseases caused by hepatitis virus, acute hepatitis, hepatocirrhosis, brain damage caused by hepatitis virus, human fulminant liver failure, sepsis, organ transplantation rejection, ischemic cardiac disease, dementia, stroke, brain impairment due to AIDS, diabetes, gastric ulcer, etc.

As a caspase inhibitor, (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide) of the following Formula 1 is attracting attention as an effective caspase inhibitor.

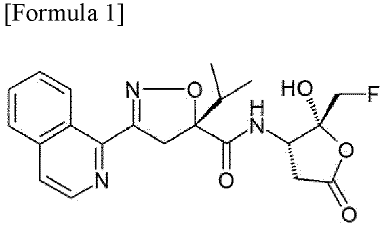

However, when the compound of Formula 1 is administered in an oral formulation, the drug delivery efficiency is poor because the compound of Formula 1 is not only exposed throughout the body but also hardly enters the articular cavity. As such, there is a need for the development of a new formulation with high drug delivery efficiency capable of maintaining the concentration of the compound of Formula 1 in the articular cavity for a long time.

### DISCLOSURE OF INVENTION

### TECHNICAL PROBLEM

Accordingly, the technical problem of the present invention is the provision of a composition for injection having high drug delivery efficiency capable of maintaining the concentration of (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide of Formula 1, which is a caspase inhibitor, in the articular cavity for a long time.

In addition, another technical problem of the present invention is the provision of a method for efficiently preparing the composition for injection.

### SOLUTION TO PROBLEM

In order to solve the above technical problem, the present invention provides a pharmaceutical composition for injection comprising a microsphere which comprises (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide of the following Formula 1, or a pharmaceutically acceptable salt or isomer thereof as an active ingredient; and poly(lactide-co-glycolide) as a biocompatible polymer:

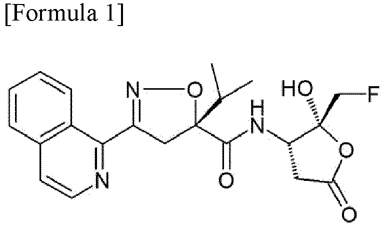

In addition, to solve another technical problem, the present invention provides a method for preparing a pharmaceutical composition for injection comprising: i) preparing a dispersed phase by dissolving (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof as an active ingredient; and poly(lactide-co-glycolide) as a biocompatible polymer in an organic solvent; ii) preparing an emulsion by adding the dispersed phase obtained in step (i) to a continuous phase; and iii) removing a solvent from the emulsion obtained in step (ii) and hardening to obtain a microsphere.

The present invention is described in detail hereinafter.

According to one aspect of the present invention, there is provided a pharmaceutical composition for injection comprising a microsphere which comprises (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide of Formula 1 (hereinafter referred to as "Compound of Formula 1"), or a pharmaceutically acceptable salt or isomer thereof as an active ingredient; and poly(lactide-co-glycolide) as a biocompatible polymer.

In the present invention, the Compound of Formula 1 may form a pharmaceutically acceptable salt. A pharmaceutically acceptable salt may include an acid-addition salt which is formed from an inorganic acid such as hydrochloric acid, sulfuric acid, nitric acid, phosphoric acid, hydrobromic acid and hydroiodic acid; an organic acid such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acid such as methanesulfonic acid, ethanesulfonic acid, benzenesulfonic acid and p-toluenesulfonic acid, which form nontoxic acid-addition salt including pharmaceutically acceptable anion. In addition, a pharmaceutically acceptable carboxylic acid salt includes the salt with alkali metal or alkali earth metal such as lithium, sodium, potassium, calcium and magnesium; salts with amino acid such as lysine, arginine and guanidine; an organic salt such as dicyclohexylamine, N-methyl-D-glucamine, tris(hydroxymethyl)methylamine, diethanolamine, choline and triethylamine. The compound of Formula 1 according to the present invention may be converted into their salts by conventional methods.

Meanwhile, since the Compound of Formula 1 according to the present invention can have an asymmetric carbon center and asymmetric axis or plane, they can exist as E- or Z-isomer, R- or S-isomer, racemic mixtures or diastereoisomer mixtures and each diastereoisomer, all of which are within the scope of the present invention.

Herein, unless indicated otherwise, the term "the Compound of Formula 1" is used to mean all the Compound of Formula 1, pharmaceutically acceptable salts and isomers thereof.

In one embodiment of the present invention, the weight ratio of the Compound of Formula 1, or a pharmaceutically acceptable salt or isomer thereof to poly(lactide-co-glycolide) may be 5 to 20 : 80 to 95. In one embodiment of the present invention, the weight ratio of the Compound of Formula 1, or a pharmaceutically acceptable salt or isomer thereof to poly(lactide-co-glycolide) may be 7 to 18 : 82 to 93.

In one embodiment of the present invention, the poly(lactide-co-glycolide) (PLGA) as a biocompatible polymer may be polymerized from lactide and glycolide by ring-opening polymerization in the presence of a catalyst. In one embodiment of the present invention, the molar ratio of lactide to glycolide of the poly(lactide-co-glycolide) may be 40:60 to 90:10. In one embodiment of the present invention, the molar ratio of lactide to glycolide of the poly(lactide-co-glycolide) may be 70:30 to 90:10. In one embodiment of the present invention, the molar ratio of lactide to glycolide of the poly(lactide-co-glycolide) may be 50:50. In one embodiment of the present invention, the molar ratio of lactide to glycolide of the poly(lactide-co-glycolide) may be 75:25. In one embodiment of the present invention, the molar ratio of lactide to glycolide of the poly(lactide-co-glycolide) may be 85:15. In one embodiment of the present invention, the poly(lactide-co-glycolide) may be used in a mixture having different molar ratios of lactide to glycolide. For example, as the poly(lactide-co-glycolide), those having a molar ratio of lactide to glycolide of 50:50 and 75:25 may be mixed and used, or those having a molar ratio of lactide to glycolide of 75:25 and 85:15 may be mixed and used.

In one embodiment of the present invention, the molecular weight of the poly(lactide-co-glycolide) may be 20 to 500 kDa. In one embodiment of the present invention, the molecular weight of the poly(lactide-co-glycolide) may be 70 to 200 kDa.

In one embodiment of the present invention, the poly(lactide-co-glycolide) may have an ester or an acid, and more preferably an ester as an end group.

In one embodiment of the present invention, the pharmaceutical composition for injection may further comprise a solvent. Examples of the solvent include, but are not limited to, water, saline or phosphate-buffered saline.

The pharmaceutical composition for injection according to the present invention may further comprise other ingredients such as a dispersing agent, a wetting agent or a suspending agent, if necessary.

Exemplary diseases that can be prevented or treated by the pharmaceutical composition for injection according to the present invention include, but are not limited to, those selected from apoptosis-associated diseases, inflammatory diseases, osteoarthritis, rheumatoid arthritis, degenerative arthritis and destructive bone disorders.

In one embodiment of the present invention, the pharmaceutical composition for injection according to the present invention may be used for the prevention, treatment or pain relief of osteoarthritis.

According to another aspect of the present invention, there is provided a method for preparing a pharmaceutical composition for injection comprising: i) preparing a dispersed phase by dissolving (R)-N-((2S,3 S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof as an active ingredient; and poly(lactide-co-glycolide) as a biocompatible polymer in an organic solvent; ii) preparing an emulsion by adding the dispersed phase obtained in step (i) to a continuous phase; and iii) removing a solvent from the emulsion obtained in step (ii) and hardening to obtain a microsphere.

In one embodiment of the present invention, the weight ratio of the Compound of Formula 1, or a pharmaceutically acceptable salt or isomer thereof to poly(lactide-co-glycolide) in step (i) may be 5 to 20 : 80 to 95. In one embodiment of the present invention, the weight ratio of the Compound of Formula 1, or a pharmaceutically acceptable salt or isomer thereof to poly(lactide-co-glycolide) in step (i) may be 7 to 18 : 82 to 93.

In one embodiment of the present invention, the molar ratio of lactide to glycolide of the poly(lactide-co-glycolide) may be 40:60 to 90:10. In one embodiment of the present invention, the molar ratio of lactide to glycolide of the poly(lactide-co-glycolide) may be 70:30 to 90:10. In one embodiment of the present invention, the molar ratio of lactide to glycolide of the poly(lactide-co-glycolide) may be 50:50. In one embodiment of the present invention, the molar ratio of lactide to glycolide of the poly(lactide-co-glycolide) may be 75:25. In one embodiment of the present invention, the molar ratio of lactide to glycolide of the poly(lactide-co-glycolide) may be 85:15. In one embodiment of the present invention, the poly(lactide-co-glycolide) may be used in a mixture having different molar ratios of lactide to glycolide. For example, as the poly(lactide-co-glycolide), those having a molar ratio of lactide to glycolide of 50:50 and 75:25 may be mixed and used, or those having a molar ratio of lactide to glycolide of 75:25 and 85:15 may be mixed and used.

In one embodiment of the present invention, the molecular weight of the poly(lactide-co-glycolide) may be 20 to 500 kDa. In one embodiment of the present invention, the molecular weight of the poly(lactide-co-glycolide) may be 70 to 200 kDa.

In one embodiment of the present invention, the poly(lactide-co-glycolide) may have an ester or an acid, and more preferably an ester as an end group.

In one embodiment of the present invention, the organic solvent in step (i) may be selected from dichloromethane, ethyl acetate, dimethyl sulfoxide, dimethylformamide, acetic acid, hydrochloric acid, methanol, ethanol, acetone, chloroform, N-methyl-2-pyrrolidone, tetrahydrofuran, methyl ethyl ketone, propyl acetate, methyl acetate and a mixture thereof. In one embodiment of the present invention, the organic solvent in step (i) may be selected from dichloromethane, ethyl acetate and a mixture thereof. In one embodiment of the present invention, the organic solvent in step (i) may be a mixture of dichloromethane and ethyl acetate. In one embodiment of the present invention, the mixing ratio of dichloromethane and ethyl acetate may be 9: 1.

In one embodiment of the present invention, the continuous phase of step (ii) may be a polyvinyl alcohol (PVA) solution. For example, 0.5% by weight polyvinyl alcohol solution, 1% by weight polyvinyl alcohol solution or 2% by weight polyvinyl alcohol solution may be used. In one embodiment of the present invention, the continuous phase of step (ii) may be adjusted to pH 3 to 5 by using an acid. In one embodiment of the present invention, the continuous phase of step (ii) may be adjusted to pH 4 by using an acid. In one embodiment of the present invention, the acid may be acetic acid.

In one embodiment of the present invention, the preparation of the emulsion in step (ii) may be carried out by a homogenizer, inkjet printing or membrane emulsification, but is not limited thereto. In one embodiment of the present invention, the membrane emulsification may be carried out with SPG (Shirasu porous glass) membrane.

### ADVANTAGEOUS EFFECTS OF INVENTION

In the present invention, by providing a pharmaceutical composition for injection in which the Compound of Formula 1-which is a caspase inhibitor-is comprised, a biodegradable sustained-release drug delivery system capable of maintaining the concentration of the Compound of Formula 1 in the articular cavity for a long time can be provided.

### BRIEF DESCRIPTION OF DRAWINGS

Figure 1 is photographs taken with a scanning electron microscope (SEM) of the microspheres prepared in Examples 1 to 3.
Figure 2 is photographs taken with an optical microscope of the microspheres prepared in Examples 4 to 23.
Figure 3 is photographs taken with a scanning electron microscope (SEM) of the microspheres prepared in Examples 24 and 25.
Figure 4 is a graph showing the density of the dispersed phase and the solubility of the Compound of Formula 1 according to the ratio of dichloromethane (DCM)/ethyl acetate (EA).
Figure 5 is results showing the morphologies and particle size distributions of the microspheres prepared in Examples 26 to 29.
Figure 6 is a graph showing the results of the *in vitro* dissolution test of the microspheres prepared in Examples 26 to 29.
Figure 7 is results showing the morphologies and particle size distributions of the microspheres prepared in Examples 30 to 32.
Figure 8 is a graph showing the results of the *in vitro* dissolution test of the microspheres prepared in Examples 30 to 32 in comparison with the results of the *in vitro* dissolution test of the microspheres prepared in Examples 26, 28 and 29.
Figure 9 is photographs taken with an optical microscope of the microspheres prepared in Examples 33 to 36.
Figure 10 represents a comparison of vertical and horizontal stirring in Experimental Example 13.
Figure 11 is a graph showing the results of the *in vitro* dissolution test according to vertical and horizontal stirring in Experimental Example 13.
Figure 12 is a graph showing the PK test results of the microspheres prepared in Example 38.
Figure 13 is graphs showing the POC (proof of concept) test results of the microspheres prepared in Example 38 and Comparative Example 1.

### MODE FOR THE INVENTION

Hereinafter, the present invention will be described in more detail through preparation examples and examples. However, these examples are only illustrative, and the scope of the present invention is not limited thereto.

### Preparation Example: Synthesis of (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide

(R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide (hereinafter referred to as "Test Compound") was prepared according to the same method as disclosed in International Publication No. WO 2006/090997.

### Examples 1 to 3: Preparation of microspheres using homogenizer

According to the compositions denoted in Table 1 below, microspheres encapsulated with the Test Compound, which are drug sustained-release carriers for articular cavity administration, were prepared.

The weight ratio of the Test Compound and PLGA was weighed at about 1:10 as represented in Table 1, and an organic solvent dichloromethane (DCM) was added thereto and stirred to prepare a dispersed phase. The PLGA used had an L/G ratio of 50:50 and a molecular weight (M.W.) of 32,000.

For a continuous phase, 50 mL of 0.5% polyvinyl alcohol (M.W. 31,000-50,000, degree of hydrolysis 87-89%) was used. The dispersed phase was put into the continuous phase, and the emulsion particle size was adjusted with a homogenizer according to rpm (8,000 to 20,500).

The prepared emulsions were stirred for 4 hours and hardened by vaporizing the organic solvent to prepare microspheres.

**[Table 1]**

| Example | PLGA MW (g/mol) | PLGA (mg) | DCM (mL) | Test Compound (mg) | PVA 0.5 wt% (mL) | Homogenizer (rpm) | Mixing time (min) | Hardening (hr) |
|---|---|---|---|---|---|---|---|---|
| Example 1 | 32K | 200.4 | 0.8 | 20.3 | 50 (about 41.5°C) | 8,000 | 5 | 4 |
| Example 2 | | 199.8 | | 20.1 | | 13,500 | | |
| Example 3 | | 199.9 | | 20.0 | | 20,500 | | |

### Experimental Example 1: Analysis of microsphere morphologies

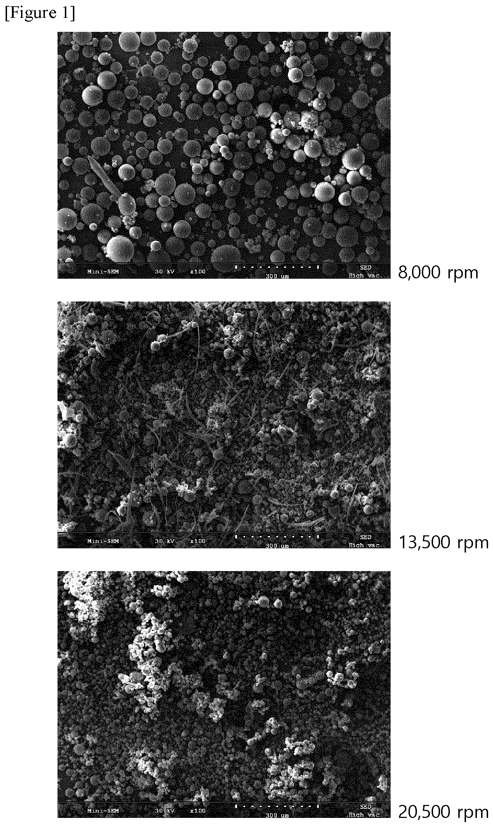

The morphologies of the microspheres prepared in Examples 1 to 3 were analyzed using scanning electron microscopy, and the results are represented in Figure 1.

As can be seen from Figure 1, the prepared microspheres showed a particle distribution of several µm to 100 µm or more in size, and the particle size tended to decrease according to the rotational speed of the homogenizer. Accordingly, it can be known that the particle size can be adjusted according to the rotational speed.

### Examples 4 to 23: Preparation of microspheres using inkjet printing

After filling the dispersed phase solution in the inkjet head, the surface of the head was wetted with the continuous phase, and spraying was performed at an appropriate frequency and flow rate.

As represented in Table 2, the PLGA ratio of the dispersed phase was 10 wt% to 33 wt%, the frequency was 0.5 kHz to 3 kHz, and the flow rate was 5 mL/min to 20 mL/min. As PLGA, 5050 DLG 4.5E (L/G ratio 50:50, M.W. 62,000, ester terminated) was used.

**[Table 2]**

| Example | PLGA content (wt%) | Flow rate (ml/min) | Frequency (kHz) |
|---|---|---|---|
| Example 4 | 10 | 20 | 0.5 |
| Example 5 | | | 0.8 |
| Example 6 | | | 1 |
| Example 7 | | | 1.5 |
| Example 8 | | | 2 |
| Example 9 | | | 3 |
| Example 10 | 30 | 10 | 0.5 |
| Example 11 | | | 0.8 |
| Example 12 | | | 1 |
| Example 13 | | | 1.5 |
| Example 14 | | | 2 |
| Example 15 | | | 3 |
| Example 16 | 32 | 10 | 0.8 |
| Example 17 | | | 1 |
| Example 18 | | | 1.5 |
| Example 19 | | | 2 |
| Example 20 | | | 3 |
| Example 21 | 33 | 5 | 0.8 |
| Example 22 | | | 1 |
| Example 23 | | | 1.5 |

### Experimental Example 2: Analysis of microsphere morphologies

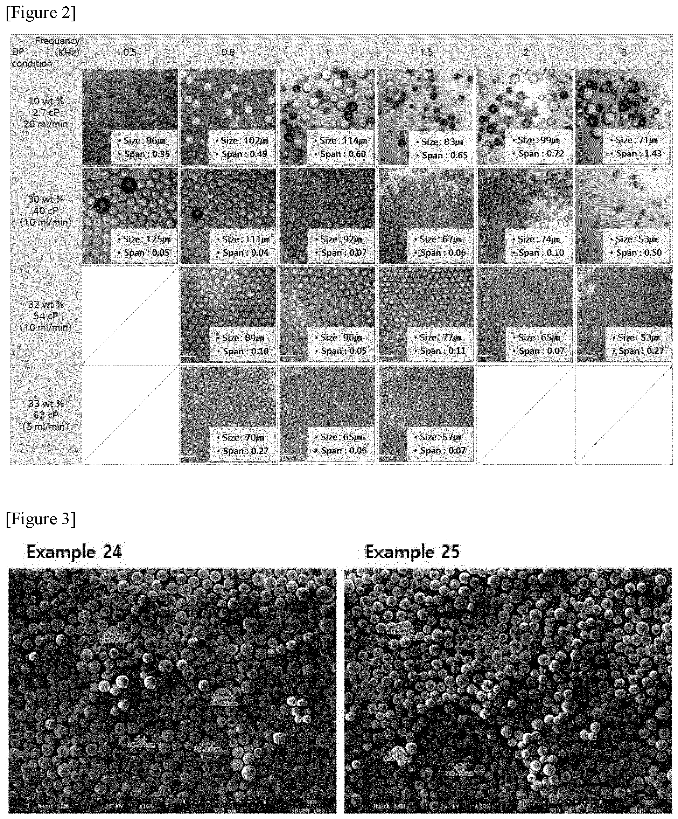

The morphologies of the microspheres prepared in Examples 4 to 23 were analyzed using an optical microscope, and the results are represented in Figure 2.

As can be seen from Figure 2, it was confirmed that the emulsion particle size was adjusted according to the frequency and flow rate. As the dispersion phase concentration (viscosity) increased, particle formation was possible in the range where the spraying speed was low, and nozzle clogging appeared. Specifically, it was thought that it would be difficult to apply in a dispersed phase with low viscosity (PLGA 10% by weight, about 3 cp or less) or high viscosity (about 80 cp or more) because of the large effect by viscosity.

### Examples 24 and 25: Preparation of microspheres using SPG membrane

Microspheres were prepared according to the compositions denoted in Table 3 below.

In the case of encapsulating the Test Compound (Example 25), the weight ratio of the Test Compound and PLGA was weighed at about 1:10 as represented in Table 3, and after adding 10 mL of the organic solvent dichloromethane (DCM), stirring was carried out to obtain a dispersed phase. The PLGA used had an L/G ratio of 50:50 and a molecular weight (M.W.) of 32,000. As a continuous phase, 150 mL of 0.5% polyvinyl alcohol (MW 31,000-50,000, degree of hydrolysis 87-89%) was used.

The emulsions were prepared using a Shirasu porous glass (SPG) device and a hydrophilic membrane with a pore size of 20 µm, and the operating pressure was 1.3 kPa to 2.1 kPa.

**[Table 3]**

| Example | PLGA MW (g/mol) | PLGA (g) | DCM (ml) | Test Compound (mg) | PVA 0.5 wt% (ml) | Pressure (kPa, outlet) | Note |
|---|---|---|---|---|---|---|---|
| Example 24 | 32K | 1.0 | 10 | - | 150 | 1.3 | Blank |
| Example 25 | | 1.0 | | 100 | | 2.1 | API added |

### Experimental Example 3: Analysis of microsphere morphologies

The morphologies of the microspheres prepared in Examples 24 and 25 were analyzed using scanning electron microscopy, and the results are represented in Figure 3.

As can be seen from Figure 3, it can be known that the size of the microspheres was about 20 µm to 60 µm, and the microspheres can be prepared into considerably uniform particles through the SPG membrane.

### Experimental Example 4: Test for API loss according to continuous phase

A dispersed phase was prepared by dissolving a certain amount of the Test Compound (API) in an organic solvent (dichloromethane, DCM), and 1 mL of the prepared dispersed phase was added to each continuous phase and then stirred to measure the amount of the API extracted into the continuous phase. The results are represented in Table 4.

**[Table 4]**

| Continuous phase composition | DW | PVA 1wt% | PVA 5wt% | 0.01 N HCl | PVA 1 wt% + 1 N HCl |
|---|---|---|---|---|---|
| Amount of API extracted into continuous phase (%) | 3.7 | 24.9 | 59.3 | 4.0 | 13.1 |

As the concentration of polyvinyl alcohol (PVA) used as a stabilizer for emulsions in the continuous phase increased, the amount of API extracted into the continuous phase increased. It was judged that the high concentration of PVA acted as a solubilizer in the continuous phase to increase the solubility of the API. In addition, it was confirmed that the extraction amount of the API decreased when 1 N HCl was added to 1% by weight of PVA according to the properties of the API showing low solubility under acidic conditions.

### Experimental Example 5: Evaluation for dispersion phase suitability

It was confirmed that precipitation of the Test Compound occurred even with a small temperature change in the dispersed phase due to the low solubility of the Test Compound. Therefore, it was tried to find a solvent suitable for use as a dispersed phase by checking the physicochemical properties of various solvents, and the solubility, emulsion stability, solubility in water, density, boiling point and suitability were tested and are represented in Table 5.

While adding a small amount of the Test Compound to each solvent (10 mL), the concentration at the point where it no longer dissolves was identified. In the case of dichloromethane (DCM) used in the dispersion phase of the existing formulation, it showed a solubility of about 20.0 mg/mL, and it was confirmed that most solvents had a solubility higher than that.

Emulsions were prepared by adding the same amount of organic solvent to the continuous phase (PVA 1 wt%), and agglomeration/phase separation of emulsions was checked over time. It was confirmed that dichloromethane, butyl acetate (BA) and methyl propionate (MP) maintained the stability of the emulsion for a considerable period of time.

The physicochemical properties of each solvent were checked through literature and the like. During the hardening process of the emulsion, a part of the organic solvent was dissolved in the aqueous phase and then evaporated into the air, so the solubility in water and boiling point were identified. In addition, if the density of the formed emulsion is too low, the emulsion floats to the surface of the continuous phase, and thus there may be a possibility of forming a polymer film on the surface. As such, the density of the solvent was identified.

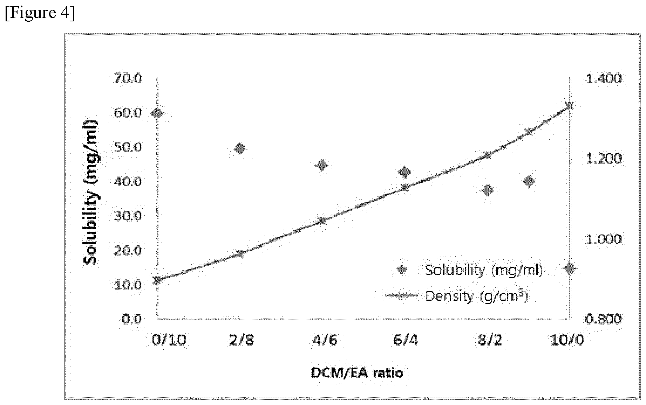

The density and solubility according to the co-solvent ratio of dichloromethane (DCM) and ethyl acetate (EA)-which are widely used solvents for microsphere preparation-were measured, and the results are represented in Figure 4.

When the density of the dispersed phase is lower than that of the continuous phase (PVA aqueous solution), the emulsion may harden on the surface of the aqueous phase, and a polymer film may be formed. Therefore, as shown in Figure 4, it can be known that DCM/EA (9: 1) co-solvent having a high density and a high solubility of the Test Compound is preferable.

**[Table 5]**

| Organic solvent | Test Compound solubility (mg/mL) | Emulsion stability (PVA 1 wt%) | Solubility in water | Density (g/cm³) | Boiling point (°C) | Suitability |
|---|---|---|---|---|---|---|
| Methylene chloride | 20 | Good | 17.5 g/L at 25°C | 1.33 | 39.6 | △△ |
| Ethyl acetate | 61.3 | Soluble | 8.7% at 20°C | 0.902 | 77.1 | △ |
| 2-Butanone | 86.5 | Bad | 275.0 g/L | 0.805 | 79.6 | X |
| Butyl acetate | 19.9 | Good | 6.8 g/L at 20°C | 0.883 | 126.1 | X |
| Ethyl formate | 48.7 | Soluble | 9% (17.78°C) | 0.917 | 54 | △ |
| 1 -Methyl-2-pyrrolidone | 87.7 | Bad | Completely | 1.028 | 202 | X |
| Methyl propionate | 56.1 | Good | 72.0 g/L at 20°C | 0.915 | 80 | ΔΔ |

### Examples 26 to 29: Preparation of microspheres (evaluation of effect according to PLGA properties)

PLGA is a copolymer of lactic acid and glycolic acid, and it has been known that decomposition and drug release are affected by the polymerization ratio (L/G ratio) and molecular weight of the two monomers. Test Compound microspheres were prepared by applying PLGA having different L/G ratios, molecular weights and end groups, and the effect of PLGA properties on drug release was evaluated.

For the preparation of microspheres, an SPG membrane device was used, and four (4) types of microspheres were prepared under the conditions according to Table 6. In addition, to evaluate the effect of PLGA properties, the composition of the dispersed phase and the continuous phase, hardening conditions and process conditions were carried out in the same manner as much as possible.

The weight ratio of the Test Compound and PLGA was weighed at about 16.7:83.3 as represented in Table 6. PLGA and organic solvent dichloromethane (DCM) were added at a weight ratio of 1:10, followed by stirring to prepare a dispersed phase.

The PLGAs used were the following four types.
a. 5050 DLG 4.5E (L/G ratio 50:50, M.W. 62,000, ester terminated)
b. 7525 DLG 4A (L/G ratio 75:25, M.W. 51,000, acid terminated)
c. 7525 DLG 4E (L/G ratio 75:25, M.W. 52,000, ester terminated)
d. 7525 DLG 8E (L/G ratio 75:25, M.W. 133,000, ester terminated)

For a continuous phase, 150 mL of 2% polyvinyl alcohol (M.W. 31,000-50,000, degree of hydrolysis 87-89%) was used, and the pH was adjusted to 3 using acetic acid. Emulsions were prepared by a membrane emulsification method, and at that time the pressure was 5.0 to 6.0 kPa and spraying was carried out at a rotational speed of 160 to 165 rpm for about 3 hours. Microspheres were prepared by removing the solvent while stirring the emulsion firstly at 35°C for 1 hour and secondly at 25°C for 15 hours.

**[Table 6]**

| Example | Example 26 | Example 27 | Example 28 | Example 29 | |
|---|---|---|---|---|---|
| PLGA | 5050 DLG 4.5E | 7525 DLG 4A | 7525 DLG 4E | 7525 DLG 8E | |
| IV (dL/g) | 0.46 | 0.38 | 0.38 | 0.79 | |
| MW (kDa) | 62 | 51 | 52 | 133 | |
| TG (°C) | 45 | - | 48.4 | - | |
| Membrane pore size | 30 µm | | | | |

| Continuous phase condition | | 150 mL | | | |
|---|---|---|---|---|---|
| | | PVA 2 wt % (pH 3.0, acetic acid) | | | |
| Dispersed phase condition | API (g) | 0.3 | | | |
| | PLGA (g) | 1.5 | | | |
| | MC (g) | 15.0 | | | |
| Process condition | Pressure (kPa) | 6.0 | 6.0 | 5.0 | 6.0 |
| | Rotational speed (rpm) | 165 | 162 | 163 | 165 |
| | Spraying time | 3 hours | | | |
| Hardening condition | First | 35°C, 1 hour | | | |
| | Second | 25°C, 15 hours | | | |

### Experimental Example 6: Analysis of microsphere morphologies

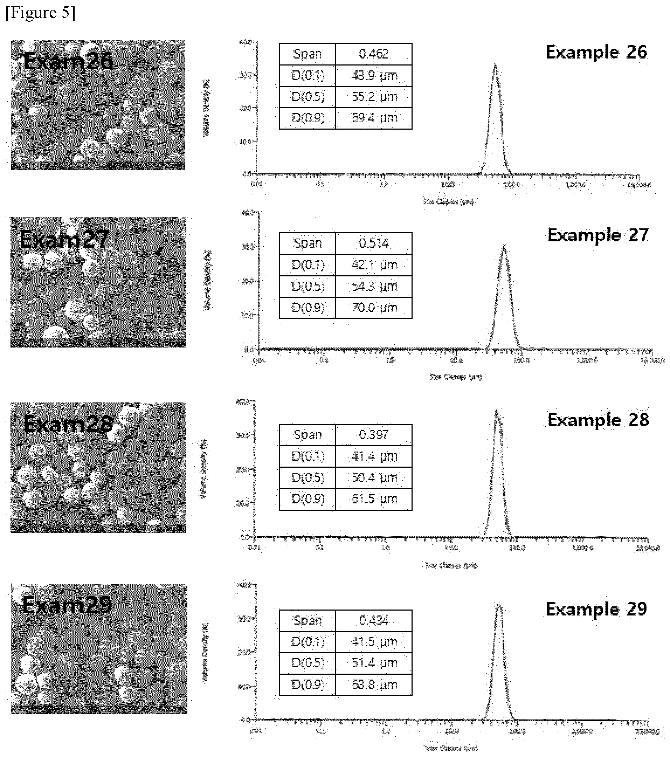

The morphologies of the microspheres prepared in Examples 26 to 29 were analyzed using scanning electron microscopy, and the particle size was measured using a particle size analyzer to determine the particle-size distribution (PSD). The results are represented in Figure 5.

As can be seen from Figure 5, it was confirmed that all the microparticles prepared in Examples 26 to 29 had smooth surfaces and spherical shape. In addition, it was confirmed that all of the microspheres prepared in Examples 26 to 29 had a size of about 40 to 70 µm and a uniform particle size distribution with a span value of 1.0 or less.

### Experimental Example 7: In vitro dissolution test of microspheres

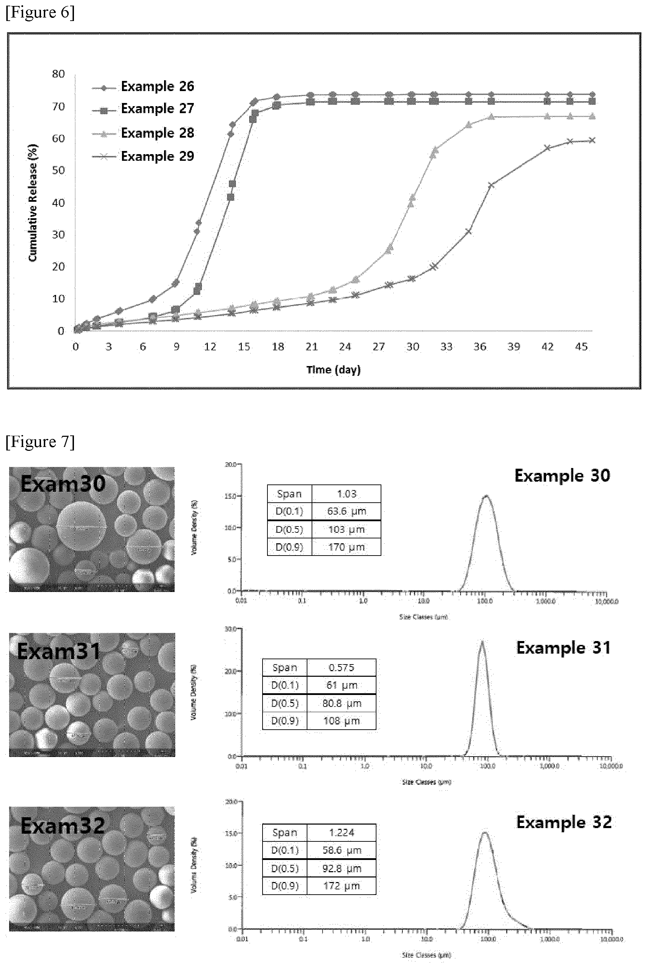

Microspheres prepared in Examples 26 to 29 were placed in a dialysis membrane, and dissolution was carried out in a tube containing 1× PBS (phosphate buffered saline). After collecting and filtering the eluate in the tube on a predetermined date, the amount of the drug released was measured by HPLC (high performance liquid chromatography). Dissolution was carried out for about 45 days, and the cumulative release was calculated by representing the percentage of the dissolution amount compared to the encapsulation amount. The results are represented in Figure 6.

It was confirmed that the release of the drug was delayed when the lactic acid ratio and intrinsic viscosity (IV) value were high. In addition, when the end group of PLGA was ester, the release delay effect was higher than that of acid, and it showed a bimodal phase in which bursting occurred at a specific point regardless of the type of PLGA. The final dissolution rate was low (around 50-70%), which was thought to be due to low stability of the Test Compound under basic conditions (eluent: 1× PBS, pH 7.4).

### Examples 30-32: Preparation of large microspheres

Microspheres larger than Examples 26, 28 and 29 were prepared. For the preparation of microspheres, an SPG membrane device was used, and three (3) types of microspheres were prepared under the conditions according to Table 7. In addition, in order to check the effect according to particle size, other conditions (dispersed phase and continuous phase composition, hardening conditions, process conditions, etc.) except for process pressure were carried out in the same manner as Examples 26, 28 and 29 as much as possible.

The weight ratio of the Test Compound to PLGA was weighed at about 16.7:83.3 as represented in Table 7. PLGA and organic solvent dichloromethane (DCM) were added at a weight ratio of 1:10, followed by stirring to prepare a dispersed phase.

The PLGAs used were the following three types.
a. 5050 DLG 4.5E (L/G ratio 50:50, M.W. 62,000, ester terminated)
b. 7525 DLG 4E (L/G ratio 75:25, M.W. 52,000, ester terminated)
c. 7525 DLG 8E (L/G ratio 75:25, M.W. 133,000, ester terminated)

For a continuous phase, 150 mL of 2% polyvinyl alcohol (M.W. 31,000-50,000, degree of hydrolysis 87-89%) was used, and the pH was adjusted to 3 using acetic acid. Emulsions were prepared by a membrane emulsification method, and at that time the pressure was 0.8 to 1.0 kPa and spraying was carried out at a rotational speed of 150 to 154 rpm for about 1 hour. Microspheres were prepared by removing the solvent while stirring the emulsion firstly at 35°C for 1 hour and secondly at 25°C for 15 hours.

**[Table 7]**

| Example | | Example 30 | Example 31 | Example 32 |
|---|---|---|---|---|
| PLGA | | 5050 DLG 4.5E | 7525 DLG 4E | 7525 DLG 8E |
| IV (dL/g) | | 0.46 | 0.38 | 0.79 |
| MW (kDa) | | 62 | 52 | 133 |
| TG (°C) | | 45 | 48.4 | - |
| Membrane pore size | | 30 µm | | |
| Continuous phase condition | | 150 mL | | |
| | | PVA 2 wt% (pH 3.0, acetic acid) | | |
| Dispersed phase condition | API (g) | 0.3 | | |
| | PLGA (g) | 1.5 | | |
| | MC (g) | 15.0 | | |
| Process condition | Pressure (kPa) | 0.8 | 0.8 | 1.0 |
| | Rotational speed (rpm) | 154 | 150 | 152 |
| | Spraying time | 1 hour | | |
| Hardening condition | First | 35°C, 1 hour | | |
| | Second | 25°C, 15 hours | | |

### Experimental Example 8: Analysis of microsphere morphologies

The morphologies of the microspheres prepared in Examples 30 to 32 were analyzed using scanning electron microscopy, and the particle size was measured by using a particle size analyzer to determine the particle-size distribution (PSD). The results are represented in Figure 7.

As can be seen from Figure 7, it was confirmed that all the microparticles prepared in Examples 30 to 32 had smooth surfaces and spherical shape. In addition, it was confirmed that the microspheres prepared in Examples 30 to 32 had around D(0.1) 58-64 µm and D(0.9) 100-170 µm, and a wide particle size distribution with a span value of 0.5-1.2.

### Experimental Example 9: In vitro dissolution test of microspheres

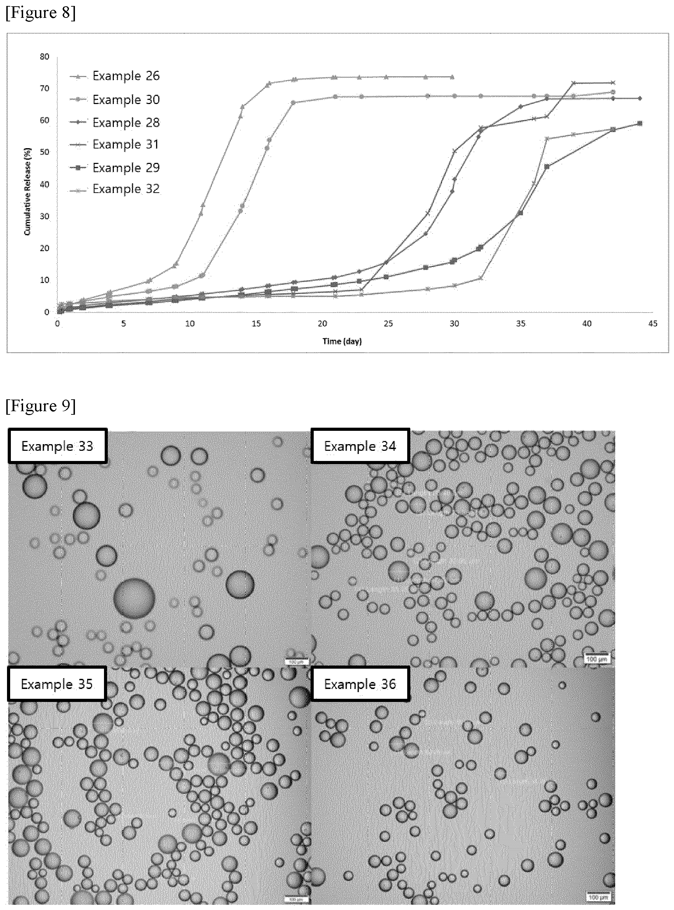

Microspheres prepared in Examples 30 to 32 were placed in a dialysis membrane, and dissolution was carried out in a tube containing 1× PBS (phosphate buffered saline). After collecting and filtering the eluate in the tube on a predetermined date, the amount of the drug released was measured by HPLC (high performance liquid chromatography). Dissolution was carried out for about 45 days, and the cumulative release was calculated by representing the percentage of the dissolution amount compared to the encapsulation amount, and then the results were compared with the *in vitro* dissolution test results of Examples 26, 28 and 29. The results are represented in Figure 8, and effects according to size were analyzed.

When the particle size of microspheres is small compared to when the particle size of microspheres is large, the drug release amount appeared relatively quickly from the beginning to the stage before bursting occurs (initial slope). Specially, when the L/G ratio of PLGA was 50:50, the bursting time was about 2 days earlier when the particle size was smaller.

From this, it can be known that when the particles are small, the surface area of the same mass is large, so the drugs on the surface of the PLGA particles are released at an early stage faster, and because there are more PLGA exposed to water, the PLGA is hydrolyzed more quickly and the time at which the drug inside is bursting could also appear sooner.

### Examples 33 to 36: Scale-up preparation of microspheres

For the preparation of microspheres, an SPG membrane device was used, and four types of microspheres were prepared under the conditions according to Table 8.

PLGA and organic solvent dichloromethane (DCM) were added at a weight ratio of 1:10, followed by stirring to prepare a dispersed phase. The PLGA used was RG504 (L/G ratio =50/50, MW=38,000-54,000, ester terminated). For a continuous phase, 5,100 mL of 0.5% polyvinyl alcohol (M.W. 31,000-50,000, degree of hydrolysis 87-89%) was used. Emulsions were prepared by a membrane emulsification method, and at that time the pressure was 0.3 to 1.1 kPa and spraying was carried out at a rotational speed of 180 rpm for about 1 hour. The emulsion was stirred at room temperature for 4 hours to remove the solvent, and then lyophilization was carried out to prepare microspheres.

**[Table 8]**

| Example | | Example 33 | Example 34 | Example 35 | Example 36 |
|---|---|---|---|---|---|
| PLGA | | RG504 (L/G=50/50, MW = 38k-54k (Da), Ester end) | | | |
| Membrane pore size | | 30 µm | | | |
| Continuous phase condition | | 5,100 mL | | | |
| | | PVA 0.5 wt% | | | |
| Dispersed phase condition | PLGA (g) | 20 | | | |
| | DCM (g) | 200 | | | |
| Process condition | Pressure (kPa) | 1.1 | 0.9 | 0.7 | 0.3 |
| | Rotational speed (rpm) | 180 | | | |
| | Spraying time | 1 hour | | | |
| Hardening | | Room temperature 4 hours (180 rpm) | | | |
| Lyophilization | | Freezing: -80°C overnight | | | |
| | | Drying: 10-20Pa 2 days | | | |

### Experimental Example 10: Analysis of microsphere morphologies

The morphologies of the microspheres prepared in Examples 33 to 36 were analyzed using an optical microscope, and the results are represented in Figure 9.

As can be seen from Figure 9, it was confirmed that the shapes of all the particulates prepared in Examples 33 to 36 were spherical, and as the spraying pressure decreased from 1.1 kPa to 0.3 kPa, the size decreased and uniform.

### Experimental Example 11: Particle size analysis

After obtaining the particle-size distribution (PSD) of the microspheres prepared in Examples 33 to 36 using a particle size analyzer, the results are represented in Table 9.

**[Table 9]**

| Example | Pressure (kPa) | Dv (10) | Dv (50) | Dv (90) | Span |
|---|---|---|---|---|---|
| Example 33 | 1.1 | 53.6 | 102 | 199 | 1.43 |
| Example 34 | 0.9 | 38.9 | 65.8 | 132 | 1.41 |
| Example 35 | 0.7 | 40.7 | 64.9 | 107 | 1.02 |
| Example 36 | 0.3 | 38.4 | 57.9 | 85.5 | 0.81 |

### Examples 37 and 38: Preparation of microspheres for POC

For the preparation of microspheres for proof of concept (POC) test, a pilot scale SPG membrane device was used, and two (2) types of microspheres were prepared under the conditions according to Table 10.

The weight ratio of the Test Compound to PLGA was weighed at about 16.7:83.3 as represented in Table 10. PLGA and organic solvent (DCM (dichloromethane) : EA (ethyl acetate) = 9 : 1) were added at a weight ratio of 1: 10 as represented in Table 10, followed by stirring to prepare a dispersed phase. The PLGAs used were RG504 (L/G ratio =50/50, MW=38,000-54,000, ester terminated) and RG756S (L/G ratio =75/25, MW=76,000-115,000, ester terminated).

For a continuous phase, 5,100 mL of 1% polyvinyl alcohol (M.W. 31,000-50,000, degree of hydrolysis 87-89%) was used, and the pH was adjusted to 4 using acetic acid. Emulsions were prepared by a membrane emulsification method, and at that time the pressure was 0.3 kPa and spraying was carried out at a rotational speed of 180 rpm for about 1 hour. The prepared emulsion was stirred at room temperature for 4 hours to remove the solvent, and lyophilization was carried out to prepare microspheres.

**[Table 10]**

| Example | | Example 37 | Example 38 |
|---|---|---|---|
| PLGA | | RG504 | RG756S |
| Membrane pore size | | 30 µm | |
| Continuous phase condition | | 5,100 mL PVA 1 wt% (pH 4.0, acetic acid) | |
| Dispersed phase condition | API (g) | 3.4 | |
| | PLGA (g) | 17 | |
| | Solvent (g) | 170 (DCM : EA = 9 : 1) | |
| Process condition | Pressure (kPa) | 0.3 | |
| | Rotational speed (rpm) | 180 | |
| | Spraying time | 1 hour | |
| Hardening | | Room temperature 4 hours (180 rpm) | |
| Lyophilization | | Freezing: -80°C overnight | |
| | | Drying: 10-20Pa 2 days | |

### Experimental Example 12: Measurement of encapsulation rate and particle size of microspheres

The encapsulation rate and particle size of the microspheres prepared in Examples 37 and 38 were measured. The encapsulation rate was measured by the following method.
1) Approximately 30 mg of microspheres loaded with the Test Compound were weighed, accurately recorded, and placed in a 50 mL volumetric flask.
2) About 1/3 of 80% acetonitrile (MeCN) was added to the volumetric flask of (1), and the microspheres loaded with the Test Compound were completely dissolved by sonication.
3) When the microspheres were completely dissolved, 80% MeCN was added up to the 50 mL mark.
4) Filtering was carred out by using a syringe filter and HPLC measurement was carried out.
5) The peak area of the Test Compound was obtained from the measured chromatogram, and this area was interpolated into the standard curve function of the Test Compound (making three or more concentrations of the Test Compound solution at an appropriate concentration and measuring them with HPLC to obtain the linear relationship of x (concentration) and y (peak area)) as the y value to obtain the x value corresponding to the concentration (mg/mL) of the Test Compound.
6) Because the volume of the solution in which the microspheres are dissolved is 50 mL, multiplying the concentration obtained in (5) by 50 gives the amount of the Test Compound (API amount) encapsulated in the microspheres.
7) The amount of the Test Compound measured in (6) was divided by the amount of the Test Compound initially added and multiplied by 100 to obtain the encapsulation rate (%).

The measured encapsulation rate and particle size of microspheres are represented in Table 11.

**[Table 11]**

| Example | Amount (g) | API | PLGA | Encapsulation rate (%) | Dx(10) | Dx(50) | Dx(90) | Span |
|---|---|---|---|---|---|---|---|---|
| Example 37 | 10 | Test Compound | RG504 | 8.2 | 34.3 | 45.2 | 59.2 | 0.551 |
| Example 38 | 10 | Test Compound | RG756S | 7.8 | 34.7 | 60.2 | 158 | 2.055 |

The encapsulation rate of the microspheres prepared in Examples 37 and 38 was about 8%. The microspheres prepared in Example 37 had an average size of 45 µm and a span of about 0.6 which were small and uniform, and the microspheres prepared in Example 38 had an average size of 60 µm and a span of about 2.1. It was confirmed that the microspheres of Example 38 had a larger size and a wider distribution than those of Example 37.

### Experimental Example 13: In vitro dissolution test of microspheres (difference between vertical and horizontal stirring)

Microspheres prepared in Examples 37 and 38 were placed in a dialysis membrane and dissolution was carried out in a tube containing 1× PBS. After collecting and filtering the eluate in the tube on a predetermined date, the amount of the drug released was measured by HPLC (high performance liquid chromatography). Dissolution was carried out for about 39 days, and cumulative release was calculated by representing the percentage of the dissolution amount compared to the encapsulation amount.

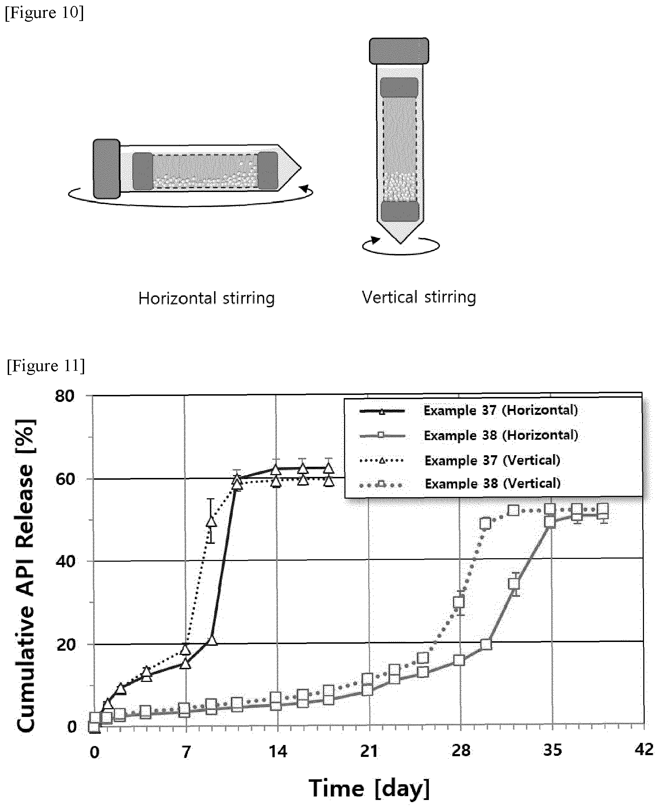

As shown in Figure 10, the tube was laid horizontally and stirred, or vertically stirred to check the difference in the stirring method. The graphs of Example 37 (horizontal) and Example 38 (horizontal) were obtained by stirring while the tube was laid horizontally, and the graphs of Example 37 (vertical) and Example 38 (vertical) were obtained by stirring the tube vertically. The comparison results are represented in Figure 11.

As can be seen from Figure 11, the Test Compound was released slightly faster in the test method in which the tube was vertically placed and stirred.

### Experimental Example 14: Pharmacokinetics (PK) test of microspheres

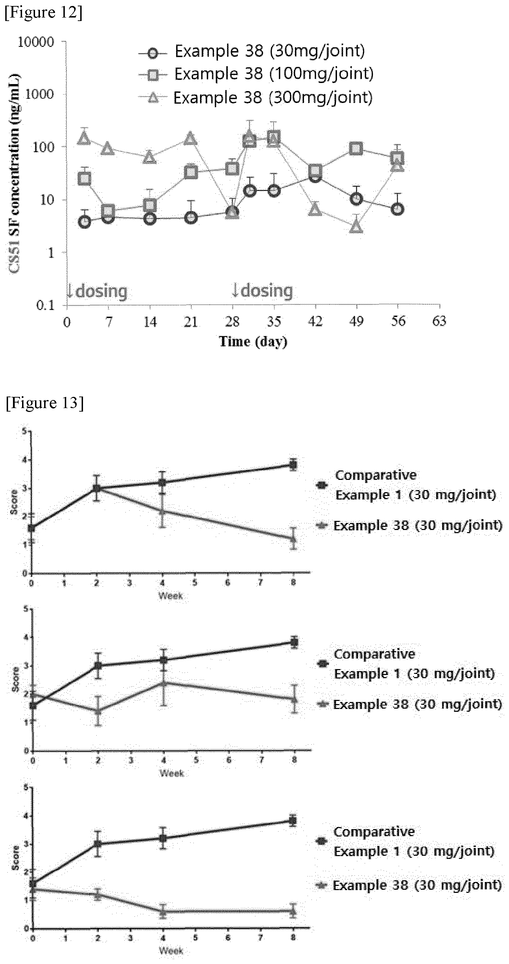

The pharmacokinetics (PK) test of the microspheres prepared in Example 38 was carried out. 30 mg, 100 mg and 300 mg of the microspheres of Example 38 were administered to the articular cavity of the dogs, and then the same amount was additionally administered once in the 4^{th} week. That is, the dosing interval was designed so that the Test Compound was present in the articular cavity for a total of 8 weeks. Synovial fluid was collected for each sampling point, and the concentration of the Test Compound in the articular cavity was analyzed. The results are represented in Figure 12.

As can be seen from Figure 12, when 30 mg, 100 mg and 300 mg of microspheres were administered to the dog articular cavity, it was confirmed that the amount of the detected Test Compound increased as the dose increased, and the dosage form continuously released the Test Compound for 3-4 weeks. When the same amount was injected once more at the 4^{th} week, it was confirmed that the Test Compound was present in the articular cavity for a total of 8 weeks.

### Comparative Example 1: Preparation of microspheres not encapsulating POC drug

A pilot scale SPG membrane device was used, and microspheres were prepared under the following conditions.

PLGA and organic solvent dichloromethane were added at a weight ratio of 1:10, followed by stirring to prepare a disperse phase. The PLGA used was RG756S (L/G ratio = 75/25, MW = 76,000-115,000, ester terminated).

As a continuous phase, 5,100 mL of 1% polyvinyl alcohol (M.W. 31,000-50,000, degree of hydrolysis 87-89%) was used. Emulsions were prepared by a membrane emulsification method, and at that time the pressure was 0.3 kPa and spraying was carried out at a rotational speed of 180 rpm for about 1 hour. The prepared emulsion was stirred at room temperature for 4 hours to remove the solvent, and lyophilization was carried out to prepare microspheres.

### Experimental Example 15: Microsphere particle size analysis

After obtaining the particle-size distribution (PSD) of the microspheres prepared in Comparative Example 1 using a particle size analyzer, the results are represented in Table 12.

**[Table 12]**

| Comparative Example | Amount (g) | API | PLGA | Dx(10) | Dx(50) | Dx(90) | Span |
|---|---|---|---|---|---|---|---|
| Comparative Example 1 | 9.6 | None | RG756S | 35.4 | 47.1 | 62.7 | 0.6 |

It was confirmed that the microspheres prepared in Comparative Example 1 had an average size of 47 µm and Span = 0.6, and thus the average size was smaller and the distribution was narrower than the microspheres prepared in Example 38 in which the average size is 60 µm and Span = 2.1.

### Experimental Example 16: POC (Proof of Concept) test

The POC experiment of the microspheres prepared in Example 38 was carried out. To evaluate the efficacy in dogs, menisectomy operation was performed, forced exercise was carried out 1 week after surgery, and POC samples were administered 2 weeks after surgery. As in Experimental Example 14, the Test Compound was administered again 4 weeks after the first administration to maintain the concentration. The efficacy was verified through walking ability evaluation for 8 weeks after the first administration. For comparison, microspheres prepared in Comparative Example 1 without drug were also administered in the same manner.

Based on the lameness score shown in Table 13, the dogs' walking ability was observed for 8 weeks and quantified as a score, and the results are represented in Figure 13.

**[Table 13]**

| Score | Observation |
|---|---|
| 0 | No observable lameness |
| 1 | Intermittent, mild weight bearing lameness with little, if any, change in gait |
| 2 | Moderate weight bearing lameness-obvious lameness with noticeable gait change |
| 3 | Severe weight bearing lameness-"toe-touching" only |
| 4 | Non-weight bearing |

As can be seen from Figure 13, administration of the microspheres of Comparative Example 1, which did not encapsulate the test compound, had no effect at all, and the operated leg could hardly stand on the floor. The lameness score of the group administered with the microspheres of Comparative Example 1 started at score 3 in 2 weeks and gradually deteriorated to score 4 in 8 weeks.

When 30 mg of the microspheres of Example 38 encapsulating the Test Compound was administered, there was no efficacy for 2 weeks after administration. However, after 2 weeks dogs-who could not walk-walked well, and after 8 weeks the lameness score was about 1, indicating that the efficacy was excellent. When 100 mg and 300 mg were administered, the efficacy came out almost immediately, and it was confirmed that the larger the dose, the faster and greater the efficacy.

## Claims

1. A pharmaceutical composition for injection comprising a microsphere which comprises (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof as an active ingredient; and poly(lactide-co-glycolide) as a biocompatible polymer.

2. The pharmaceutical composition for injection according to Claim 1, wherein a weight ratio of (R)-N-((2S,3 S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof to poly(lactide-co-glycolide) is 5 to 20 : 80 to 95.

3. The pharmaceutical composition for injection according to Claim 2, wherein a weight ratio of (R)-N-((2S,3 S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof to poly(lactide-co-glycolide) is 7 to 18 : 82 to 93.

4. The pharmaceutical composition for injection according to Claim 1, wherein a molar ratio of lactide to glycolide of the poly(lactide-co-glycolide) is 40:60 to 90:10.

5. The pharmaceutical composition for injection according to Claim 4, wherein a molar ratio of lactide to glycolide of the poly(lactide-co-glycolide) is 70:30 to 90:10.

6. The pharmaceutical composition for injection according to Claim 1, wherein a molecular weight of the poly(lactide-co-glycolide) is 20 to 500 kDa.

7. The pharmaceutical composition for injection according to Claim 6, wherein a molecular weight of the poly(lactide-co-glycolide) is 70 to 200 kDa.

8. The pharmaceutical composition for injection according to Claim 1, wherein an end group of the poly(lactide-co-glycolide) is ester or acid.

9. The pharmaceutical composition for injection according to Claim 8, wherein an end group of the poly(lactide-co-glycolide) is ester.

10. The pharmaceutical composition for injection according to Claim 1, which further comprises a solvent.

11. The pharmaceutical composition for injection according to Claim 10, wherein the solvent is water, saline or phosphate-buffered saline.

12. The pharmaceutical composition for injection according to Claim 1, which is for the prevention or treatment of a disease selected from apoptosis-associated diseases, inflammatory diseases, osteoarthritis, rheumatoid arthritis, degenerative arthritis and destructive bone disorders.

13. The pharmaceutical composition for injection according to Claim 12, which is for the prevention, treatment or pain relief of osteoarthritis.

14. A method for preparing a pharmaceutical composition for injection comprising:
i) preparing a dispersed phase by dissolving (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof as an active ingredient; and poly(lactide-co-glycolide) as a biocompatible polymer in an organic solvent;
ii) preparing an emulsion by adding the dispersed phase obtained in step (i) to a continuous phase; and
iii) removing a solvent from the emulsion obtained in step (ii) and hardening to obtain a microsphere.

15. The method for preparing a pharmaceutical composition for injection according to Claim 14, wherein a weight ratio of (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof to poly(lactide-co-glycolide) in step (i) is 5 to 20 : 80 to 95.

16. The method for preparing a pharmaceutical composition for injection according to Claim 15, wherein a weight ratio of (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazole-5-carboxamide, or a pharmaceutically acceptable salt or isomer thereof to poly(lactide-co-glycolide) in step (i) is 7 to 18 : 82 to 93.

17. The method for preparing a pharmaceutical composition for injection according to Claim 14, wherein a molar ratio of lactide to glycolide of the poly(lactide-co-glycolide) is 40:60 to 90:10.

18. The method for preparing a pharmaceutical composition for injection according to Claim 17, wherein a molar ratio of lactide to glycolide of the poly(lactide-co-glycolide) is 70:30 to 90:10.

19. The method for preparing a pharmaceutical composition for injection according to Claim 14, wherein a molecular weight of the poly(lactide-co-glycolide) is 20 to 500 kDa.

20. The method for preparing a pharmaceutical composition for injection according to Claim 19, wherein a molecular weight of the poly(lactide-co-glycolide) is 70 to 200 kDa.

21. The method for preparing a pharmaceutical composition for injection according to Claim 14, wherein an end group of the poly(lactide-co-glycolide) is an ester or an acid.

22. The method for preparing a pharmaceutical composition for injection according to Claim 21, wherein an end group of the poly(lactide-co-glycolide) is an ester.

23. The method for preparing a pharmaceutical composition for injection according to Claim 14, wherein the organic solvent of step (i) is selected from dichloromethane, ethyl acetate, dimethyl sulfoxide, dimethylformamide, acetic acid, hydrochloric acid, methanol, ethanol, acetone, chloroform, N-methyl-2-pyrrolidone, tetrahydrofuran, methyl ethyl ketone, propyl acetate, methyl acetate and a mixture thereof.

24. The method for preparing a pharmaceutical composition for injection according to Claim 23, wherein the organic solvent of step (i) is a mixture of dichloromethane and ethyl acetate.

25. The method for preparing a pharmaceutical composition for injection according to Claim 24, wherein a mixing ratio of dichloromethane and ethyl acetate is 9:1.

26. The method for preparing a pharmaceutical composition for injection according to Claim 14, wherein the continuous phase of step (ii) is a polyvinyl alcohol (PVA) solution.

27. The method for preparing a pharmaceutical composition for injection according to Claim 14, wherein the continuous phase of step (ii) is adjusted to pH 3 to 5 by using an acid.

28. The method for preparing a pharmaceutical composition for injection according to Claim 27, wherein the acid is acetic acid.

29. The method for preparing a pharmaceutical composition for injection according to Claim 14, wherein the preparation of the emulsion in step (ii) is carried out by a homogenizer, inkjet printing or membrane emulsification.

30. The method for preparing a pharmaceutical composition for injection according to Claim 29, wherein the membrane emulsification is carried out with SPG (Shirasu porous glass) membrane.
